# EUROPEAN PATENT SPECIFICATION

(11) **EP 1 879 028 B1**
(45) Date of publication and mention of the grant of the patent: **20.08.2008**
(21) Application number: 07112174.3
(22) Date of filing: 10.07.2007
(51) Int. Cl.: G01N 33/52, G01N 33/543

(54) **Use of albumin, bovine, p-aminophenyl n-acetyl ß-d glucosaminide as a positive control line in an immunoassay device**
Verwendung von Albumin, Bovin, P-Aminophenyl-N-Acetyl-ß-D-Glucosaminid als Positivcontrolle für eine Immuntestvorrichtung
Utilisation d'albumine, P-aminophényl N-acétyl ß-D glucosaminide bovine en tant que contrôle positif pour dispositif de dosage immunologique

(30) Priority: 12.07.2006 US 807089 P; 11.08.2006 US 463903
(43) Date of publication of application: 16.01.2008
(73) Proprietor: Becton, Dickinson & Company, Franklin Lakes, NJ 07417-1880 (US)
(72) Inventor: Lovell, Stephen J., Baltimore, MD 21239 (US); Kopher, Kenneth A., Baltimore, MD 21239 (US)
(74) Representative: von Kreisler Selting Werner

(56) References cited:
- WO-A-20/06013329
- US-A- 5 356 782
- US-A1- 2003 211 634
- US-B1- 6 824 997

## Description

### FIELD OF THE INVENTION

The invention relates to control reagents utilized in immunoassays that detect the presence or absence, or determine the amount of a particular analyte of interest present in a sample.

### BACKGROUND OF THE INVENTION

In vitro diagnostic (IVD) tests have revolutionized the rapid analysis of analytes, and allow for a simple and cost effective detection method for a myriad of moieties including proteins such as enzymes and hormones, drugs and drug metabolites, antibodies, and nucleic acids. Many of these tests are based on immunoassays that combine the principles of chemistry and immunology to provide for quantitative and qualitative analyses of target analytes. The basic principle of these assays is the detection of an analyte-receptor reaction.

In recent years, immunoassays have evolved from expensive and complex procedures requiring calibrated machinery and skilled technicians for operation to more simplistic designs such as dip-sticks and test strips using relatively inexpensive binder support mediums that are easily operated by anyone because they only require the operator to follow a simple series of directions. Today, immunoassays based on these binder support mediums offer rapid results and increasing sensitivity for the detection of a large number of analytes of interest. For example, immunoassay devices have been developed to detect the presence of *Streptococcus pyogenes*, otherwise known as Group A Streptococcus (GAS), the bacteria that causes illnesses such as strep throat.

While there are many permutations to the design of the binder support medium based immunoassay, one common design, known as a "sandwich assay," involves detecting the analyte of interest following its binding to a labeled receptor (or "tracer"), and the necessary separation of the free labeled receptor from the bound labeled receptor. The analyte of interest is generally contained or placed in a sample that is then added to the immunoassay. As the sample interacts with the active reagents in the immunoassay, immunological or chemical reactions may occur that ultimately allow for the detection of the presence or amount of the analyte of interest.

These chemical reactions in sandwich assays occur as the sample flows through or across the device. The analyte, if present in the sample, binds with a receptor capable of detection, usually an antibody that recognizes the analyte of interest, and is capable of being linked or bound to a label (or "tracer") moiety that can be detected, either by an operator or by a machine. The labeled receptor is generally located within the membrane or one of the membranes of the immunoassay device.

As the analyte-labeled receptor complex flows across or through the membrane or membranes, it eventually comes into contact with a detection zone on a binder support medium of the immunoassay that comprises an immobilized capture receptor or ligand known as a binder, where it is bound. The presence or amount of the analyte of interest is determined through inspection of the detection zone, where the presence of the analyte is indicated generally by a specific visually detectable signal.

Because of the several chemical and immunological reactions that occur in using an assay, some devices further include an additional reaction step, which is generally referred to as a control reaction that produces a control signal, to verify to the operator that the sample has been analyzed. In some immunoassay devices, the control signal alerts the operator that the sample has flowed through the detection zone, indicating that the detection zone may be examined, with limited or no information regarding the efficacy of the potential ligand-receptor interaction. In other devices, the control signal conveys to the operator that the reaction conditions in the device and surrounding environment are appropriate for the detection of an analyte, if present, in a sample. Regardless of the type of control signal, if the control is not positive, i.e., does not appear, then the operator of the device is alerted that the assay did not function as expected. Therefore, the lack of a control signal is an indication that the results of the assay may not be reliable.

Ideally, an immunoassay device would utilize a comprehensive control signal to verify the efficacy of the assay. The term "comprehensive" in this context refers to control signals that verify not only whether or not the sample has passed through the detection zone, but also whether or not the conditions in the environment allow the necessary chemical and immunological reactions to occur to detect the analyte, if present, in a sample. Preferably, the control signal is one that utilizes chemical and immunological reactions that mimic the interactions and reactions on which the device is relying in detecting the presence or amount of the analyte of interest. For example, a number of assays have a control zone, located downstream of the detection zone, that comprises an immobilized form of the analyte of interest. Therefore, when unbound labeled receptors flow past the detection zone, they are capable of binding to the analyte in the control zone, assuming the conditions of the assay are appropriate, and can generate a signal. This comprehensive control signal will verify to the operator not only that the sample has passed through the detection zone and on to the control zone, but also that the device was used under conditions that will support the binding of the analyte of interest, if present, by the labeled receptor.

Certain analytes of interest, however, cannot be immobilized readily, and therefore do not function well as control reagents. For example, as disclosed in U.S.-A-5,877,028 to Chandler et al. ("the '028 patent"), most carbohydrates, including bacterial carbohydrates such as Group A Streptococcus group-specific carbohydrates, may be difficult to affix to a binder support medium using currently known techniques. The '028 patent discloses, as an alternative to a control zone comprising the analyte of interest, a control zone comprising anti-species antibodies that bind to the antibody that forms the receptor portion of the labeled receptor. Anti-species antibodies are simply antibodies that are capable of binding to an antibody of a different animal. These anti-species antibodies can be immobilized in the control zone of an assay device and bind to the unbound labeled receptors. The labeled receptors are then capable of displaying a signal verifying that the sample has passed through the detection zone. For example, the '028 patent describes the use of a rabbit antibody to a bacterial carbohydrate found in GAS bacterium as a receptor, and a goat antibody to the rabbit antibody as the control reagent.

Unlike control zones comprising an immobilized analyte of interest, the signal generated using anti-species antibodies will not necessarily verify that conditions support the binding of labeled receptors to the analyte of interest, because the binding interaction of the anti-species antibody and labeled receptor may be different from that of the analyte and labeled receptor.

One of the more common and significant analytes of interest that is difficult to immobilize is a GAS group-specific carbohydrate antigen that contains β-N-Acetyl-D-Glucosamine attached to a poly-rhamnose backbone. GAS are gram-positive pathogenic bacteria that may cause serious infections associated with strep throat, tonsillitis, septicemias, and scarlet fever and are a common cause of illness in the elderly, children, and other susceptible populations. Therefore, easily identifying contaminated areas containing GAS so that exposure to the bacteria may be reduced is highly desirable. Furthermore, because the symptoms associated with GAS infection overlap with other pathogenic causes that respond to different therapeutic treatments, it is important to identify individuals with GAS infections quickly so that proper therapeutic remedies may be prescribed.

Given the importance of accurate results, however, it also is desirable to have immunoassays that measure the presence or amount of analytes of interest with reliable controls. Currently, assay devices testing for the presence of bacterial carbohydrates in a sample, such as GAS group-specific carbohydrates, do not use the bacterial carbohydrates themselves as control reagents because the bacterial carbohydrates are difficult to immobilize on binder support mediums. Therefore, these devices must contain other control reagents that may not give a true indication of the accuracy of the assay results.

Thus, there is a need in the art for immunoassay devices that are capable of displaying a comprehensive control signal when assaying for bacterial carbohydrates. These comprehensive control signals should verify to the operator that the sample has passed through the detection zone and on to the control zone, and, importantly, that the device was used under conditions that support the binding of the labeled receptors and analyte of interest, if present.
US-A-5,356,782 and US-A-2003/0211634 disclose an immuno assay device according to the preamble of claim 1.

### SUMMARY OF THE INVENTION

The present invention provides immunoassay devices, kits and methods for displaying a comprehensive control signal when determining the presence or amount of a bacterium in a sample. The comprehensive control signals of the present invention utilize analogs to the assayed analyte of interest, wherein the analogs provide an immobilizable reagent comprising the same or similar labeled receptor binding regions as those of the analyte of interest. The use of an analog with the same or similar labeled receptor binding region to that of the bacterial analyte provides the same or similar immunological type of reaction that is used to detect the analyte in the generation of a control signal. Thus, a positive control signal generated in an immunoassay of the present invention verifies to the operator that the conditions of the assay environment, such as the integrity of the immunological activity of the labeled receptor, support the chemical and immunological reactions that generate accurate test results for the analyte of interest.

In one embodiment of the present invention, the device tests a sample for the presence or amount of a bacterium, wherein the analyte of interest is a bacterial carbohydrate, and wherein the control reagent of the device comprises an analog of the bacterial carbohydrate being assayed for, in particular the device tests a sample for the presence or amount of the GAS (hereinafter referred to as the bacterium for which it is tested), wherein the analyte of interest is a GAS group-specific carbohydrate antigen that comprises β-N-Acetyl-D-Glucosamine attached to a poly-rhamnose backbone, and wherein the control reagent of the device comprises Albumin, Bovine, p-Aminophenyl N-Acetyl β-D Glucosaminide, an analog of β-N-Acetyl-D-Glucosamine.

The present invention allows for operators to test a sample for the specific bacterium by identifying the presence of an analyte, i.e., the bacterial carbohydrate, in a sample, while also allowing the operator to determine whether or not the assay provides a reliable indication of the presence or amount of the analyte. The invention may provide for an immunoassay device with a more accurate and informative control signal when compared to devices currently known in the art that utilize control signals with different labeled receptor binding regions than the analyte of interest. Given the widespread use of immunoassays, and the importance of testing for bacteria such as GAS, wherein the analytes of interest may be carbohydrates, the present invention may fulfill a need in the art by providing a true internal control that validates the immunological reactivity of the analytes and labeled receptors used in the device.

In one embodiment, a sample containing or lacking a bacterium can be prepared and then applied to the immunoassay device wherein the sample wets labeled receptors contained in the device. In certain embodiments, the sample may be prepared prior to application of the sample to the assay by mixing the sample with a reagent. The reagent may allow for the separation of bacterium components, providing an analyte of interest such as a bacterial carbohydrate for analysis.

Following application of the sample to the assay, the analyte of interest, if present in the sample, may interact with the labeled receptor to form an analyte-labeled receptor complex. In certain non-limiting embodiments, the labeled receptor may comprise an antibody capable of recognizing and binding to an antigenic determinant, also known as an eptiope, on the analyte of interest, the antigenic determinant being the region on the analyte where the receptor portion of the labeled receptor is capable of binding. This analyte-labeled receptor complex can then flow through a detection zone and a control zone.

The detection zone may be an area on the binder support medium wherein a binder has been immobilized, the binder being capable of recognizing and binding the analyte-labeled receptor complex. As the sample comes into contact with the detection zone, the analyte-labeled receptor complex, if present, may bind to the immobilized binder, and become trapped within the detection zone. Because the labeled receptor also contains a detectable label, the trapped complex may be detected visually by the operator or by using a machine that can analyze the label on the labeled receptor in the detection zone. Labeled receptor moieties that are not bound to the analyte may be cleared from the detection zone through the flow of the sample towards the control zone.

The control zone of the present invention comprises an analog to the bacterial carbohydrate . The analog comprises Albumin, Bovine, p-Aminophenyl N-Acetyl β-D Glucosaminide which has the same or similar labeled receptor binding region as the bacterial carbohydrate, so that the labeled receptor is capable of binding the analog in a binding interaction that mimics the interaction with the analyte. In certain embodiments of the present invention, the receptor portion of the labeled receptor may be an antibody capable of recognizing and binding the same or similar antigenic determinant on the analog that is present on the analyte. Because the labeled receptor also contains a detectable label, the immobilized analog-labeled receptor complex may be detected visually by the operator or by using a machine that can analyze the label on the labeled receptor in the control zone.

After the sample has cleared the detection zone in the first portion of the binder support medium and the control zone in the second portion, the operator can observe the result of the assay. If the assay is one with a visually detectable label, then the operator may simply view the detection zone and control zone to analyze the results. If the control zone displays a signal, then the operator may conclude that the sample has migrated through the detection zone and to the control zone, and that the conditions of the immunoassay effectively provide for the necessary chemical and immunological reactions to occur, i.e., the operator may rely on the presence or amount of a signal in the detection zone as an indication of the presence or amount of bacteria in the sample. If, however, the control zone does not display a signal, then the operator should not rely on the presence or amount of a signal in the detection zone to provide any information regarding the presence of a particular bacterium in the sample.
The present invention further relates to the immuno assay kit as defmed in claim 12, and the methods of claim 17 and 18.
Preferred embodiments of the invention are apparent from the dependent claims.

### BRIEF DESCRIPTION OF THE FIGURES

Specific exemplary embodiments of the present invention will now be described by way of example only and with reference to the accompanying drawings.

**FIG. 1** is a schematic of the chemical structure of β-N-Acetyl-D-Glucosamine attached to a poly-rhamnose backbone.

**FIG. 2** is a schematic of the chemical structure of the β-N-Acetyl-D-Glucosamine analog Albumin, Bovine, p-Aminophenyl N-Acetyl β-D Glucosaminide (NAGBSA).

**FIG. 3** is a schematic of the assay device according to one exemplary embodiment of the invention illustrating a binder support medium comprising labeled receptor, a detection zone, and a control zone.

**FIG. 4** is a schematic of the assay device according to one exemplary embodiment of the invention, wherein the invention further comprises optional components including a tracer pad, neutralizing pad, sample pad, and a sump.

**FIG. 5** is a schematic of the immunoassay device according to one exemplary embodiment of the invention wherein a detection zone is located within a first portion of the binder support medium, and a control zone is located within a second portion of the binder support medium, the first portion being vertically juxtaposed with a tracer pad and the second portion being downstream from the area of contact of the first portion with the tracer pad.

**FIG. 6** is a schematic of the immunoassay device according to one exemplary embodiment of the invention wherein a tracer pad is vertically juxtaposed with a detection zone, which is vertically juxtaposed with a control zone.

**FIG. 7** is a schematic of the assay device according to one exemplary embodiment of the invention further comprising a housing for the device with apertures for adding the sample to the device, and for viewing the signals generated in the detection and control zones.

**FIGS. 8a****-c** depict three schematics of the assay device according to exemplary embodiments of the invention, illustrating three different combinations of possible signals displayed by the detection and control zones.

**FIG. 9** is a schematic of a flow diagram according to one exemplary embodiment of the invention wherein a method for detecting a bacterium in a sample and displaying a comprehensive control signal is illustrated.

**FIGS. 10a****-b** depict an illustration of a device constructed according to an exemplary embodiment of the invention.

### DETAILED DESCRIPTION

### Definitions of key terms

The term "sample" generally refers to anything that may contain a bacterium. The sample may be a biological sample, such as a biological fluid or a biological tissue. Examples of biological fluids include urine, blood, plasma, serum, saliva, semen, stool, sputum, cerebral spinal fluid, tears, ocular lens fluid, sweat, milk, ascites fluid, synovial fluid, peritoneal fluid, transdermal exudates, pharyngeal exudates, bronchoalveolar lavage, tracheal aspirations, cerebrospinal fluid, cervical mucus, vaginal or urethral secretions, mucus, amniotic fluid or the like. Fluid homogenates of cellular tissues such as, for example, hair, skin and nail scrapings, meat extracts and skins of fruits and nuts can also be considered biological fluids.

Biological tissues can be single cells or aggregates of cells, usually of a particular kind together with their intercellular substance that form one of the structural materials of a human, animal, plant, bacterial, fungal or viral structure, including connective, epithelium, muscle and nerve tissues. Examples of biological tissues include organs, tumors, lymph nodes, arteries and individual cell(s).

The term "sample" may also include water, food products, soil extracts, solids, surface swabs or wipes, and the like.

The sample can be used as obtained directly from the source or following a pretreatment so as to modify its character. The sample can be prepared in any convenient medium that does not interfere with the assay.

The sample can be treated prior to its application on the immunoassay device. Methods of treatment can involve, but are not be limited to, filtration, distillation, separation, concentration, inactivation of interfering components, and the like, as well as the addition of reagents. Methods utilized for the selection and pretreatment of biological, industrial, and environmental samples prior to testing are generally known by one of ordinary skill in the art.

The term "labeled receptor binding region," as used herein, generally refers to a region on an analyte or analog wherein the receptor portion of a labeled receptor is capable of binding.

The term "antigenic determinant," as used herein, generally refers to an epitope that is recognized by an antibody.

The term "analog," as used herein, generally refers to a reagent comprising a domain or region that mimics the characteristics of a labeled receptor binding region of an analyte of interest, such labeled receptor binding region being recognized by a particular receptor portion of a labeled receptor utilized in the assay to detect the analyte of interest.

The term "similar," as used herein, in the context of labeled receptor binding regions and antigenic determinants, generally refers to a region or binding motif on the analyte and analog that comprises sufficient similarity so that a receptor portion of a labeled receptor is capable of binding both the analog and analyte regions.

The term "Group A Streptococcus (GAS)," as used herein, refers to *Streptococcus pyogenes,* a gram-positive, nonmotile, nonsporeforming coccus that occurs in chains or in pairs of cells. GAS may cause serious infections associated with strep throat, tonsillitis, septicemias, and scarlet fever. The backbone structure of all GAS species comprises a group-specific carbohydrate antigen that contains β-N-Acetyl-D-Glucosamine attached to a poly-rhamnose backbone.

The term "comprehensive control" as used herein, refers to a control signal that verifies to an operator that the conditions of the assay provide for the necessary chemical and immunological reactions to detect the analyte of interest, if present, in a sample. The control signal may utilize chemical and immunological reactions that mimic the interactions and reactions on which the device is relying on in detecting the presence or amount of the analyte of interest.

### Detailed Description of the Invention

The present invention provides immunoassay devices, kits and methods as referred to in the claims for displaying a comprehensive control signal when testing a sample for the presence or amount of a bacterium in a sample. To test for the presence of a bacterium in the sample devices of embodiments of the invention test for the presence of a bacterial carbohydrate analyte of interest known to be contained in the bacterium. The comprehensive control signals utilizes an analog to the bacterial carbohydrate as control reagent, wherein the analog may be an immobilized reagent comprising Albumin, Bovine, p-Aminophenyl N-Acetyl β-D Glucosaminide. A principal advantage of using as a control reagent such an analog (i.e. with the same or similar labeled receptor binding regions to that of the analyte of interest) is that the same immunological-type of reactions used in detecting the analyte of interest will be used in generating a control signal. Thus, a positive control signal generated verifies to the operator that the sample has passed through the immunoassay, and that the conditions of the immunoassay support the chemical and immunological reactions that generate accurate test results, such as the integrity of the immunological activity of the labeled receptors.

In particular embodiments, the invention provides a device that may test a sample for the presence or amount of a bacterium, the device comprising:
a) a receptor moiety attached to a label moiety thereby forming a labeled receptor, wherein the receptor moiety is capable of binding with a bacterial carbohydrate analyte of interest; and
b) a binder support medium comprising a detection zone and a control zone;
wherein the detection zone provides for the detection of the analyte of interest, or derivative or homologue thereof, and the control zone is in fluid communication with the detection zone, the control zone comprising, in an immobilized form, an analog of the analyte of interest, wherein the analog comprises Albumin, Bovine, p-Aminophenyl N-Acetyl β-D Glucosaminide

In the embodiments of the invention , the device tests a sample for the presence and amount of a GAS bacterium, wherein the analyte of interest is a bacterial carbohydrate found in the GAS bacterium, and the control reagent is an analog of the analyte of interest. In the particular embodiment, the analyte of interest is a GAS group-specific carbohydrate antigen that contains β-N-Acetyl-D-Glucosamine attached to a poly-rhamnose backbone, and the control reagent is Albumin, Bovine, p-Aminophenyl N-Acetyl β-D Glucosaminide (NAGBSA), an analog of β-N-Acetyl-D-Glucosamine.

The detection zone of the invention may be capable of displaying a detectable signal, indicating the presence or amount of a particular analyte of interest in the sample. The detection zone may comprise an immobilized binder, capable of binding with the analyte of interest. The immobilized binder may be a ligand capable of binding an analyte of interest. Alternatively, it may be a ligand capable of binding an analyte-labeled receptor complex.

The control zone of the invention may be capable of displaying a detectable signal, verifying whether or not the sample has passed through the detection zone and reached the control zone, and whether or not the conditions of the immunoassay support the chemical and immunological reactions that generate accurate test results.

In exemplary embodiments, the immunoassay device may further comprise a sample pad, a sump, a neutralizing pad, a support housing, and/or a tracer pad. If the device comprises a tracer pad, the tracer pad may comprise a labeled receptor. If present, the support housing of the invention may comprise apertures that can allow access to the sample pad or tracer pad, where applicable, for the application of the sample. In addition, the housing may comprise apertures to allow visual access to the detection zone and control zone, wherein an operator can analyze the signals displayed in the detector and control zones to determine both the presence or amount of the bacterial carbohydrate in the sample, and whether or not the sample reached the control zone and whether or not the conditions of the immunoassay provided for the chemical and immunological reactions that would generate accurate test results.

The invention is not limited to any particular arrangement of the component parts. For example, a sample pad, neutralizing pad, tracer pad, binder support medium, and sump may be placed in lateral juxtaposition with one another. In such an embodiment, the sample may flow across the device by capillary action. In another example, a sample pad, neutralizing pad, tracer pad, binder support medium, and sump may be placed in vertical juxtaposition with one another, wherein the sample may flow through the device using gravitational forces, capillary forces, or both. These and other arrangements are generally known by one of ordinary skill in the art.

The invention is not limited to the type of analysis. For example, the analysis of the bacterial carbohydrate may be qualitative, semi-quantitative, or quantitative. Qualitative, semi-quantitative, and quantitative assays are known to one of ordinary skill in the art. Semi-quantitative or quantitative analysis of the concentration or amount of a bacterial carbohydrate in a sample requires the use of a calibration mechanism. For example, the calibration mechanism may be a known amount of the bacterial carbohydrate. The calibrated analog may be placed on the binder support medium in a separate zone (calibration zone) from the detection zone, wherein the tracer pad if present, comprises an amount of labeled receptor that is capable of binding to the bacterial carbohydrate in the calibration zone. To determine semi-quantitative or quantitative amounts, the intensity of label in the detection zone is compared to the intensity of label in the calibration zone. Such calibration mechanisms are generally known by one of ordinary skill in the art.

Utilization of the invention can allow for a sample containing or lacking a bacterial carbohydrate analyte of interest to be prepared and applied to the device, interacting with a mobile labeled receptor comprising i) a label moiety capable of detection attached to ii) a receptor, wherein the receptor is capable of binding to a labeled receptor binding region contained in the analyte. In certain embodiments, the receptor utilized in the invention may be an antibody to the analyte of interest, or a derivative or homologue thereof

When the sample comes into contact with the labeled receptor, the labeled receptor may be bound to the analyte of interest, if present in the sample, and may form a mobile analyte-labeled receptor complex. The sample may then flow to a detection zone and a control zone located on a binder support medium. The detection zone may comprise an immobilized binder that is capable of binding the analyte-labeled receptor complex. In certain embodiments, the immobilized binder utilized in the invention may be an antibody to the analyte of interest, capable of binding the analyte on the same or different antigenic determinant from where the analyte is bound with the labeled receptor. The detection zone may allow for the concentration of the analyte-labeled receptor complex, wherein the label provides an indication of the presence or amount of the analyte.

Labeled receptor that remains unbound in the assay may flow with the sample to the control zone portion of the binder support medium. The control zone may comprise immobilized analog to the analyte. In certain exemplary embodiments, the sample may then flow to a sump pad in fluid communication with the binder support medium, wherein the sump prevents the sample from flowing over the control zone again. The immobilized analog in the control zone may bind with the previously unbound labeled receptors, according to the same labeled receptor binding region as in the formation of the analyte-labeled receptor complexes. The concentration of labeled receptor bound to the analog in the control zone may verify to an operator of the device that the sample has reached the control zone, and the conditions present in the assay allow for the analyte to bind with the labeled receptors. This verification informs the operator that the presence or amount of a signal in the detection zone will correspond with a presence or amount of the analyte of interest in the sample.

One aspect of the invention provides a method of testing a sample for the presence or amount of a bacterium as described in claim 17.

According to the embodiment of the invention, the method determines the presence or amount of GAS bacterium in a sample, the analyte of interest is the bacterial carbohydrate comprising β-N-Acetyl-D-Glucosamine attached to a poly-rhamnose backbone, which is found in GAS bacterium, and the control zone comprises NAGBSA, an analog of β-N-Acetyl-D-Glucosamine.

According to the invention, the method determines the presence or amount of a GAS bacterium in the sample, obtained by swabbing the back of a throat that may contain a bacterial carbohydrate found in GAS bacterium. In certain embodiments, the step of preparing the sample may comprise mixing the sample with an acidic or enzymatic reagent, wherein the reaction between the reagent and the sample may release any analyte contained in the sample and enable it to bind with antibodies in the device. Acidic and enzymatic reagents capable of reacting with the sample as described are well-known to those skilled in the art.

In an exemplary embodiment, a sample obtained by means such as swabbing the back of a throat may be prepared by steps comprising admixing 5.0M sodium nitrite with 0.03M citric acid in a container, placing the sample in the container, squeezing the sample to expel as much liquid as possible from the sample, and adding the contents of the container to an assay device according to an exemplary embodiment of the invention.

Referring now to the drawings, in which like numerals represent like elements throughout the several Figures, aspects of the invention and the illustrative operating environment will be described. The Figures, while representative of certain exemplary embodiments of the invention, are not intended to limit the invention in any way.

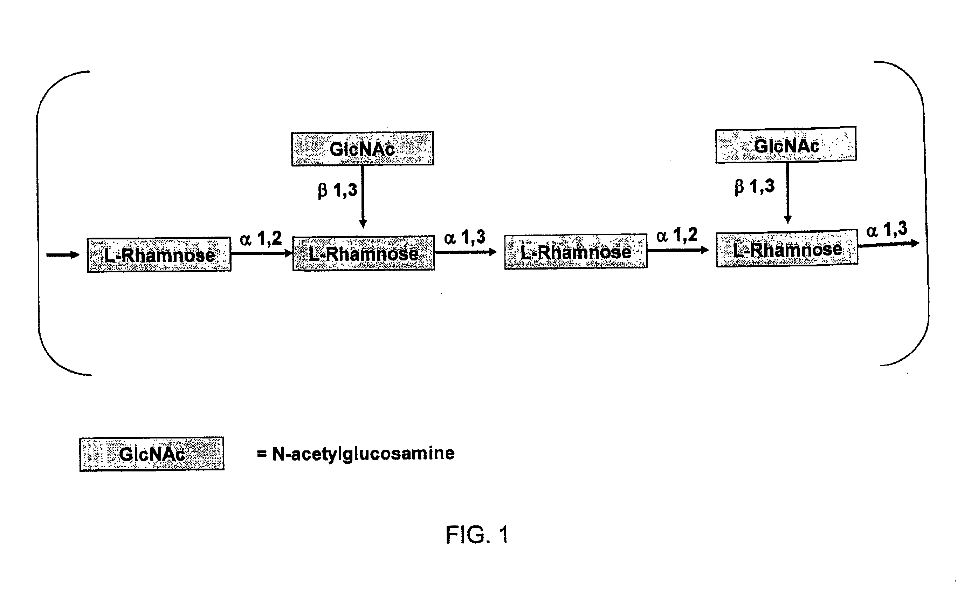

**FIG. 1** is a schematic of the chemical structure of β-N-Acetyl-D-Glucosamine attached to a poly-rhamnose backbone

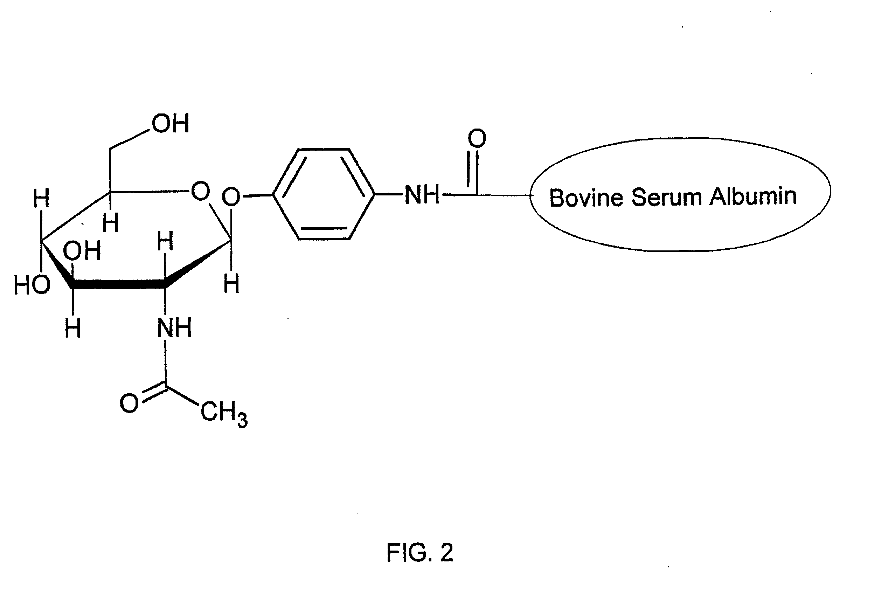

**FIG. 2** is a schematic of the chemical structure of the β-N-Acetyl-D-Glucosamine analog Albumin, Bovine, p-Aminophenyl N-Acetyl β-D Glucosaminide (NAGBSA).

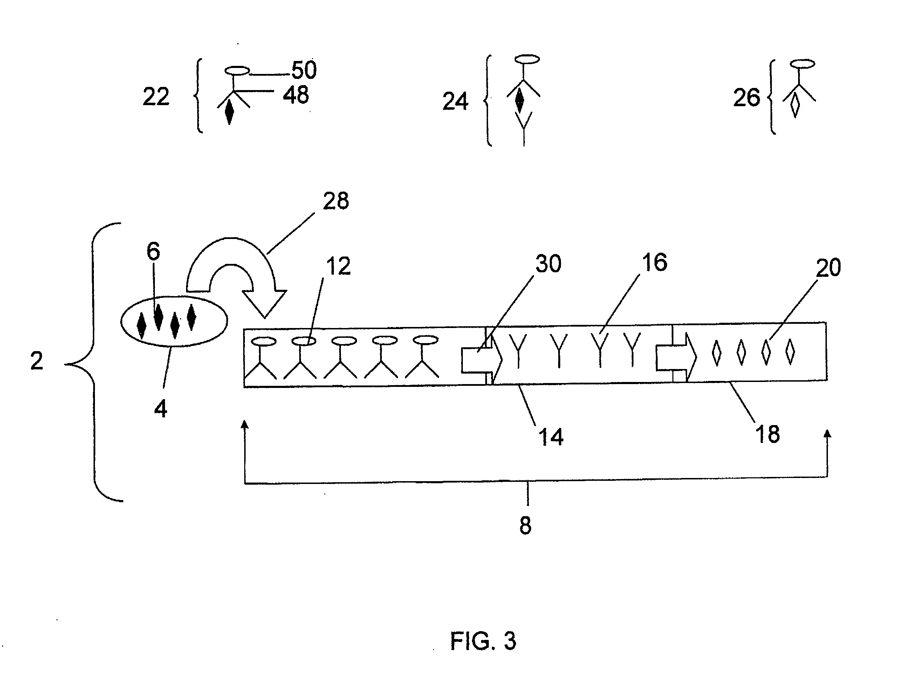

**FIG. 3** illustrates a schematic of one exemplary embodiment of an immunoassay device **2** that may be utilized to detect the presence or amount of a bacterial carbohydrate analyte of interest **6** in a sample **4.** In the illustrative embodiment, the device **2** is depicted as comprising a binder support medium **8** that comprises labeled receptor **12,** a detection zone **14,** and a control zone **18.** The labeled receptor **12** may be mobile and may comprise i) a receptor **48,** and ii) a detectable label **50** that serves as a reporter or tracer moiety attached to the receptor **48.** The attachment may be by covalent or non-covalent binding, but the method of attachment is not important to the invention. The label **50** allows the analyte-specific labeled receptor **12** to be detected, if bound and immobilized, in the detection zone **14.**

The receptor **48** portion of the labeled receptor **12** may be capable of specifically binding or complexing with the analyte of interest **6.** The analyte of interest comprises **6** β-N-Acetyl-D-Glucosamine attached to a poly-rhamnose backbone, wherein an antibody specific for that antigen may be used as the receptor **48,** or immunologically reactive fragments of the antibody, such as F(ab')2, Fab or Fab' may be used as the receptor **48.** These receptors **48** coupled to the label **50** may then bind to the analyte **6** if present in the sample **4** as the sample **4** passes through the labeled receptors **12,** and form an analyte-labeled receptor complex **22.** These analyte-labeled receptor complexes **22** may then be carried into the detection zone **14** on the binder support medium **8** by fluid flow **30** through the device **2.** When the analyte-labeled receptor complex **22** reaches the detection zone **14** it may be captured by an immobilized binder **16.**

The label **50** of the analyte-specific labeled receptor **12** may be any one of a wide variety of detectable labels known to one of ordinary skill in the art. The label **50** attached to the receptor **48** may be any substance which is capable of detection by visual or instrumental means. Various labels **50** suitable for use in the invention include labels that are detectable through either chemical or physical means. Such labels **50** may include, but are not limited to, enzymes and substrates, chromogens, catalysts, fluorescent compounds, chemiluminescent compounds, and radioactive labels. Other suitable labels **50** include particulate labels such as colloidal metallic particles such as gold, colloidal non-metallic particles such as selenium or tellurium, dyed or colored particles such as a dyed plastic or a stained microorganism, colored organic polymer latex particles and liposomes, colored beads, polymer microcapsules, sacs, erythrocytes, erythrocyte ghosts, or other vesicles containing directly visible substances, and the like.

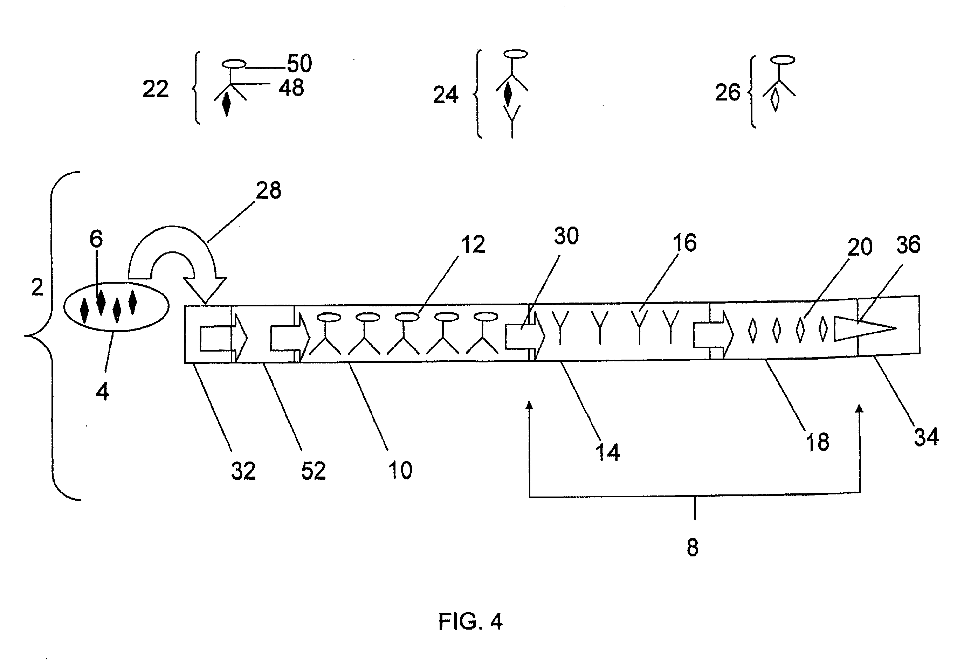

As shown in **FIG. 4****,** in certain embodiments, the device **2** may further comprise a tracer pad **10,** wherein the labeled receptors **12** are located on the tracer pad **10** instead of on the binder support medium **8.** The tracer pad **10** may be in fluid communication with a binder support medium **8.** After interaction with the labeled receptors **12** in the tracer pad **10,** the sample **4** may flow **30** from the tracer pad **10** into the detection zone **14** portion of the binder support medium **8.**

In exemplary embodiments, the detection zone **14** on the binder support medium **8** may comprise an immobilized capture reagent **16,** known as a binder, capable of binding the analyte-labeled receptor complex **22,** rendering the complex **22** immobile on the medium **8.** By "immobilized," it is generally meant that the binder **16,** once on the binder support medium **8,** may not be capable of substantial movement to positions elsewhere within the binder support medium **8.** Thus, the analyte-labeled receptor complex **22** may be trapped at the detection zone **14** through the binding of the binder **16** to the complex **22,** thereby forming a bound analyte-labeled receptor complex **24.**

The immobilized binder **16** of certain embodiments of the invention may include any moiety or compound capable of binding the analyte-labeled receptor complex **22,** analyte of interest **6,** or similar detectable complex contemplated in the type of assay employed. Because one determinant of the analyte **6** may be occupied by the labeled receptor **12** in forming the analyte-labeled receptor complex **22,** the binder **16** may consist of any ligand capable of binding to another determinant of the analyte **6.** Alternatively, the binder **16** may consist of any ligand capable of binding to the same determinant of the analyte **6** as the labeled receptor **12.** Specific binding reagents useful with the invention are known by one of ordinary skill in the art and are generally readily identifiable. In certain embodiments, the device **2** may test for the presence of the GAS bacterium, the analyte of interest **6** is the bacterial carbohydrate comprising β-N-Acetyl-D-Glucosamine attached to a poly-rhamnose backbone, which is found in GAS bacterium, and the immobilized binder **16** may be an antibody specifically directed at the β-N-Acetyl-D-Glucosamine.

Because the binder support medium **8** of the device **2** is preferably chemically inert, it can be activated at the detection zone **14** where it is desired to immobilize a specific binding reagent **16.** Various methods can render the binder reagent **16** immobilized according to the particular chemical nature of the binder support medium **8** material. The immobilized binder **16** may be supported on the binder support medium **8** in a manner which immobilizes the binder **16.** The binder **16** can be immobilized to the binder support medium **8** directly or indirectly. Direct attachment methods may include adsorption, absorption and covalent binding such as by use of (i) a cyanogen halide, e.g., cyanogen bromide or (ii) by use of glutaraldehyde. Other methods may include treatment with Schiff bases and borohydride for reduction of aldehydic, carbonyl and amino groups. DNA, RNA and certain antigens may be immobilized against solvent transport by baking onto the binder support medium **8.**

Alternatively, it may be preferred to retain or immobilize the binder reagent **16** on the binder support medium **8** material indirectly through the use of insoluble microparticles to which the binder reagent **16** has been attached. The methods of attaching an reagent to the microparticles encompass both covalent and non-covalent mechanisms including adherence, absorption, or adsorption. Microparticles are generally known by one of ordinary skill in the art. For example, microparticles may be selected from any suitable type of particulate material composed or polystyrene, polymethylacrylate, polyacrylamide, polypropylene, latex, polytetrafluoroethylene, polyacrylonitrile, polycarbonate, glass or similar material.

In certain embodiments, the binder **16** may be deposited singly or in various combinations on or in the detection zone **14** of the binder support medium **8** in a variety of configurations to produce different detection or measurement formats. Such configurations and measurement formats are generally known by one of ordinary skill in the art.

Following interaction with the detection zone **14,** the sample **4** may flow **30** away from the detection zone **14** to the control zone **18.** The control zone **18** may be juxtaposed and in fluid communication with the detection zone **14.** The control zone **18** may be capable of verifying to the operator that the sample **4** has flowed **30** to the control zone **18,** and thus through the detection zone **14** because the detection zone **14** precedes the control zone **18.**

Significantly, the control zone **18** of the present invention also verifies that the performance of the assay was within acceptable conditions when used, because the control zone **18** contains, in immobilized form, the analog **20** to the analyte of interest **6** as a control reagent. The analog **20** which comprises NAGBSA may be immobilized using any of the techniques for immobilizing binders **16,** as described above. The immobilized analog **20** reacts with labeled receptor **12** that did not bind the analyte of interest **6** in the tracer pad **10,** thereby forming an analog-labeled receptor complex **26.** The label portion of the analog-labeled receptor complex **26** may display a signal capable of being detected visually or with a machine. A signal in the control zone **18** is indicative that the conditions of the assay provide for the binding of the labeled receptors **12** to the analyte of interest **6.**

In exemplary embodiments, the analyte of interest **6** is a GAS group-specific carbohydrate antigen that contains β-N-Acetyl-D-Glucosamine attached to a poly-rhamnose backbone. In these embodiments, the control zone **18** comprises an analog **20** of β-N-Acetyl-D-Glucosamine, such as NAGBSA, in an immobilized form. The signal generated in the control zone **18** may verify to the operator that the conditions of the assay provide for the binding of the labeled receptors **12** to the β-N-Acetyl-D-Glucosamine. NAGBSA used in the control zone **18** may be obtained from vendors known to those skilled in the art. When supplied from such vendors, the NAGBSA may be lyophilized, which makes it stable when stored at temperatures under 0°C. The NAGBSA may then be hydrated and aliquoted, and then stored at a temperature under 0°C.

In preparation for application of the analog **20** to the control zone **18** of an assay device **2,** a suitable stable spotting solution may then be prepared, comprising the analog **20** in a buffer. The solution may then be applied to the control zone **18** of the binder support medium **8** by a suitable spray applicator and dried for an appropriate amount of time.

The type of binder support medium **8** that may be utilized in the invention includes, but is not limited to, substrate materials having capillarity and the capacity for chromatographic solvent transport of non-immobilized reagents and reactive sample components by means of a selected chromatographic solvent. The binder support medium **8** of the assay device **2** of the invention may be any suitably absorbent, porous or capillary possessing material through which a sample **4** containing the analyte of interest 6 may be transported by a wicking action.

**FIG. 4** depicts the invention further comprising additional elements. In exemplary embodiments of the invention, the assay device **2** may further comprise a sample pad **32** juxtaposed and in fluid communication with a neutralizing pad **52.** The sample **4** may be applied **28** to any area of the sample pad **32.** The sample **4** may flow **30** across or through the sample pad **32** to the juxtaposed neutralizing pad **52.**

In certain embodiments, as shown in **FIG. 4****,** the assay device **2** may further comprise a neutralizing pad **52** that is juxtaposed and in fluid communication with the sample pad **32** and the tracer pad **10.** The neutralizing pad **52** may comprise a base substance that may neutralize any acidic reagent used in preparing the sample **4** for application **28** to the device **2.** The neutralizing base may be necessary to prevent damage to the labeled receptors **12** and immobilized binders **16** in the device **2.** The sample **4** may flow **30** across or through the neutralizing pad **52** to the juxtaposed tracer pad **10,** wherein it may interact with the labeled receptor **12** contained therein.

**FIG. 4** further shows an exemplary embodiment of the invention, with the added element of a sump **34** juxtaposed and in fluid communication with the binder support medium **8.** The sample **4,** following contact with the detection zone **14** and subsequent contact with the control zone **18,** may be absorbed **36** by the sump **34.** This absorption **36** may prevent the sample **4** from flowing back over the control zone **18** and detection zone **14.** The sump **34** may have absorbent capacity sufficient to contain all liquid volumes used during the assay. The sump **34** may comprise any material capable of absorbing excess liquid. Examples of such materials include cotton fiber, tissue, and absorbent paper. Other examples of suitable absorbent materials capable of acting as a sump **34.**

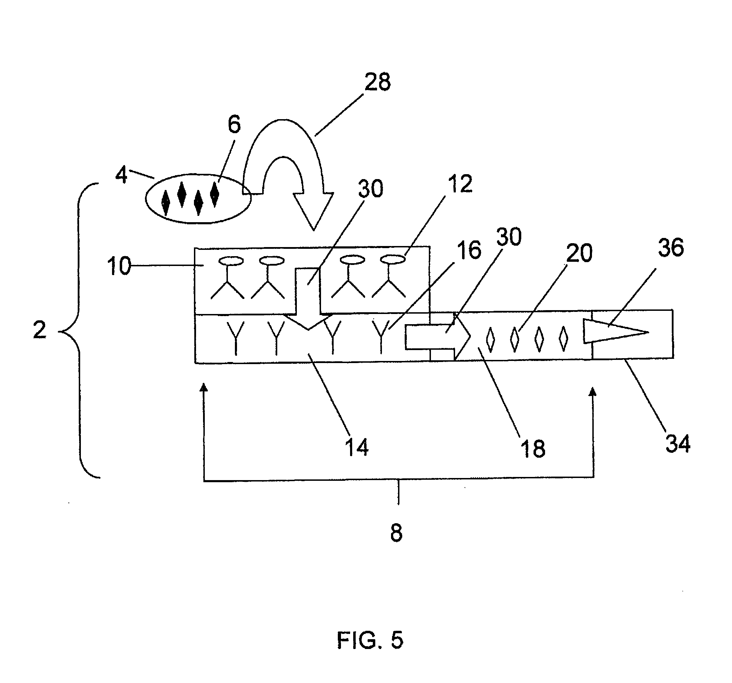

**FIG. 5** illustrates an alternative embodiment of the device **2** wherein a tracer pad **10** comprising a labeled receptor **12** is vertically juxtaposed with, and in fluid communication with, the first portion of the binder support medium **8** comprising the detection zone **14.** The second portion of the binder support medium **8** comprising the control zone **18** is in fluid communication with the detection zone **14,** but is not vertically juxtaposed with the tracer pad **10.** In this embodiment, the sample **4** may be applied **28** to the tracer pad **10,** where the labeled receptors **12** may bind with any analyte of interest **6** present in the sample **4.** The sample 4 may then flow **30** vertically towards the detection zone **14,** using gravitational and/or capillary forces, where the immobilized binders **16** will bind the analyte-labeled receptor complexes **22,** which may then display a signal. The sample **4,** including the unbound labeled receptors **12,** may then flow **30** laterally towards the control zone **18,** where the unbound labeled receptors **12** may bind with the immobilized analog **20,** and display a signal.

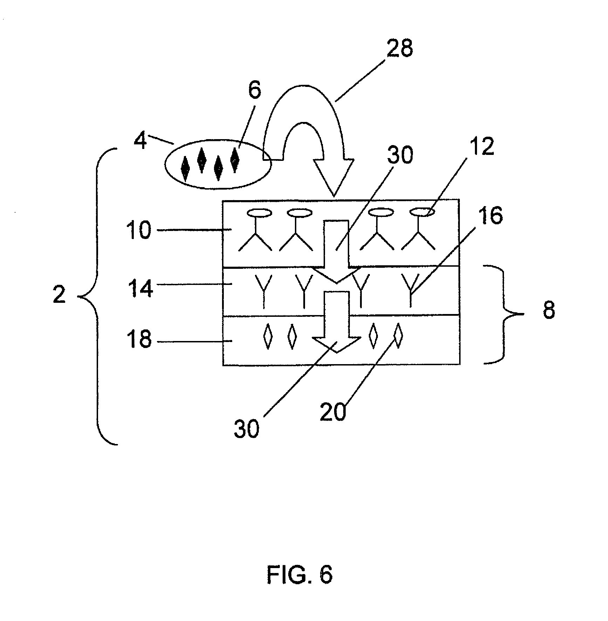

**FIG. 6** illustrates yet another embodiment of the invention, in which the device **2** may utilize vertical flow and may not utilize lateral flow. In such embodiments, an optional tracer pad **10** comprising a labeled receptor **12** may be vertically juxtaposed and in fluid communication with the detection zone **14,** and the detection zone **14** may be vertically juxtaposed and in fluid communication with the control zone **18.** In such embodiments, the sample **4** may be applied **28** to the tracer pad **10,** and may flow **30** vertically to the detection zone **14** and then flow **30** vertically to the control zone **18.**

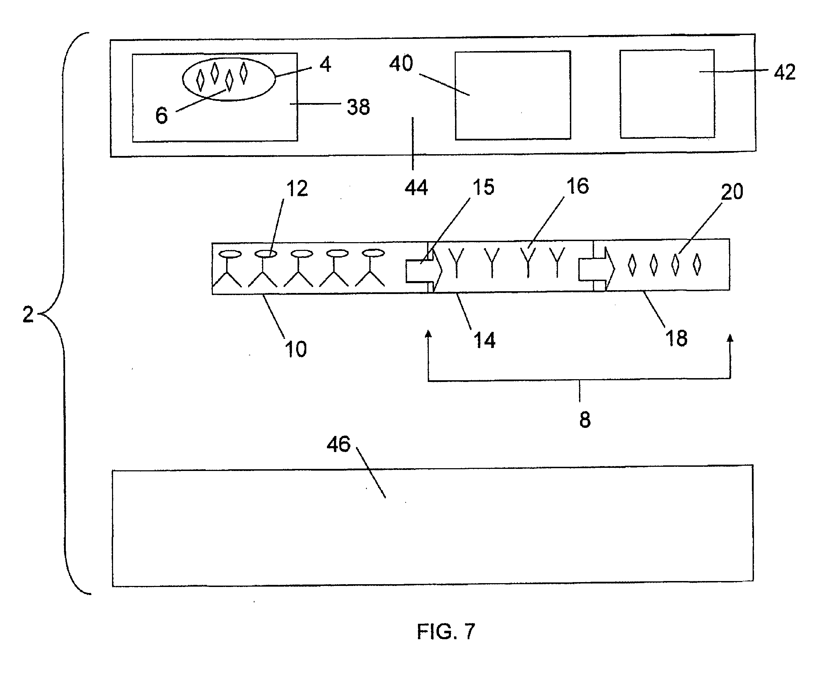

**FIG.** 7 is a schematic illustrating an exemplary embodiment wherein the device **2** is placed between an upper housing **44** and a lower housing **46.** The upper housing unit **44** may comprise an aperture **38** for access to the tracer pad **10.** The upper housing unit **44** may further comprise two apertures, an aperture **40** for visual access to the detection zone **14** and an aperture **42** for visual access to the control zone **18.** Alternatively, in other exemplary embodiments, the apertures **40, 42** for visual access to the detection zone **14** and/or control zone **18** may be placed on the lower housing unit **46.**

In one exemplary embodiment, the size of the upper housing **44** and lower housing **46** may allow for convenient handling and packaging of the device **2.** The upper housing **44** and lower housing 46 of the invention may be made of plastic, glass or other suitably rigid material. The upper housing **44** and lower housing **46** may serve other functions as well, including providing a handle or displaying information such as bar codes, fluorescent marks, or colored marks that may aid in the calibration of the assay. The upper housing **44** and lower housing **46** material may be in sheet or roll form, and may be manufactured from an opaque plastic sheet material of appropriate color, thickness, and rigidity.

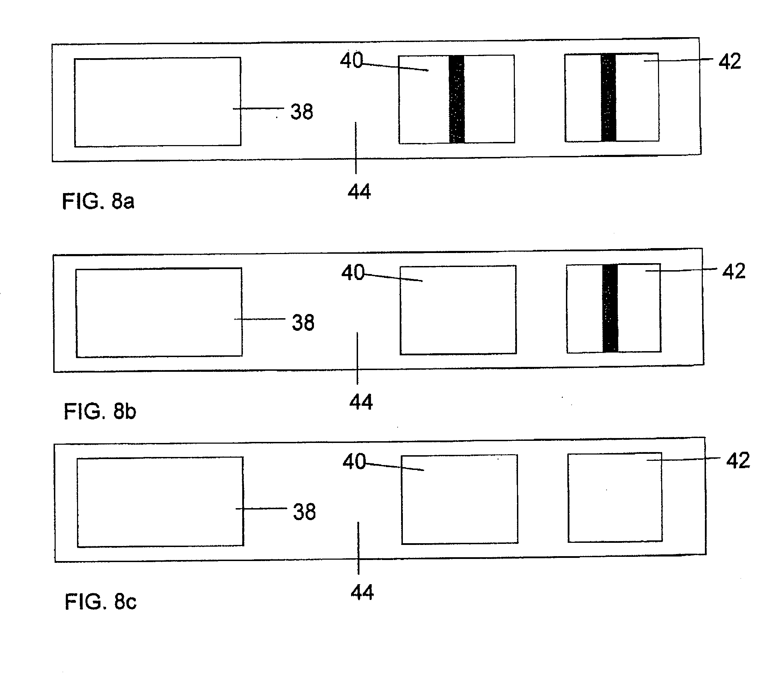

**FIGS. 8a****-c** illustrate three exemplary views of the invention in an exemplary embodiment. Each figure depicts a view of the upper housing 44 of the device **2,** after sufficient time has elapsed following the application **28** of the sample **4** to the device **2** through the aperture **38** with access to the tracer pad **10.** **FIG.** 8a shows a signal generated in the detection zone **14** that may be seen by the operator through the aperture **40** providing visual access to the detection zone **14.** **FIG. 8a** further shows a signal generated in the control zone **18** that can be seen by the operator through the aperture **42** providing visual access to the control zone **18.** Where, as in **FIG. 8a****,** the operator can detect signals generated in both the detection **14** and control **18** zones, the operator may conclude that the sample **4** has passed through the detection zone **14** and reached the control zone **18,** that the conditions in the immunoassay provided for the binding of the analyte of interest **6** and labeled receptors **12,** and that the analyte of interest **6** is present in the sample **4.**

**FIG. 8b** shows no signal generated in the detection zone **14** that can be seen through the corresponding aperture **40,** but shows a signal generated in the control zone **18.** Where, as in **FIG. 8b****,** the operator cannot detect a signal in the detection zone **14,** but can detect a signal in the control zone **18,** the operator may conclude that the sample **4** has passed through the detection zone **14** and reached the control zone **18** and that the conditions in the immunoassay provided for the binding of the analyte of interest **6** and labeled receptors **12.** The operator may further conclude that analyte **6** is not present in the sample **4,** due to a lack of signal in the detection zone **14.**

**FIG. 8c** shows no signal generated in the detection zone **14** nor in the control zone **18** that can be seen through the corresponding apertures **40, 42.** Where, as in **FIG. 8c****,** the operator cannot detect signals in either zone **14, 18,** the operator may not conclude that the sample **4** has passed through the detection zone **14** nor that the conditions in the environment immunoassay provided for the binding of the analyte of interest **6** and labeled receptors **12.** Therefore, the operator may not make any conclusion regarding the presence or amount of analyte **6** in the sample **4.**

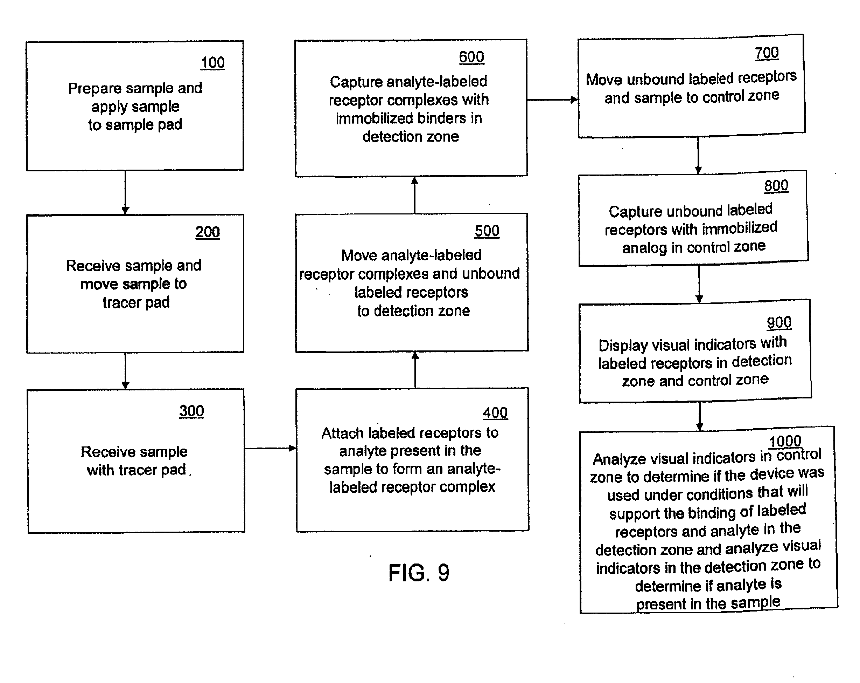

**FIG. 9** is a flow chart illustrating a method for performing an analysis for the presence or amount of a bacterial carbohydrate analyte of interest in a sample and for determining, from the control signal, whether or not to rely on a detection signal generated by the assay. The illustrative flow chart describes one use of an exemplary embodiment of the performance of an assay utilizing the device of the present invention. The flow chart described may be modified for certain alternative embodiments.

In step **100,** initiate the process by preparing a sample and applying it to the optional sample pad of the assay device. In step **200,** the sample pad receives the sample applied thereto, and the sample moves through lateral flow to an optional tracer pad. In step **300,** the tracer pad receives the sample from the sample pad. In step **400,** attach the labeled receptor capable of binding to an analyte of interest to the analyte, if present in the sample, to form an analyte-labeled receptor complex. In step **500,** move the analyte-labeled receptor complex and unbound labeled receptor to the detection zone of the binder support medium through lateral flow from the tracer pad, wherein the detection zone is juxtaposed and in fluid communication with the tracer pad. In step **600,** capture the analyte-labeled receptor complex with the immobilized binder of the detection zone. In certain embodiments, a portion of the analyte-labeled receptor may not bind with the immobilized binder of the detection zone. In such embodiments, these unbound complexes may move towards the control zone, along with the sample and unbound labeled receptor. In step **700,** move the sample and unbound labeled receptor to the control zone of the binder support medium through flow from the detection zone, wherein the control zone is juxtaposed and in fluid communication with the detection zone. In step **800,** capture the unbound labeled receptor with immobilized analog in the control zone. In step **900,** display visual indicators with the labeled receptors now immobilized in the detection zone and control zone. In step **1000,** analyze visual indicator in the control zone to determine if the sample has reached the detection and control zones, and whether or not the conditions of the immunoassay support the chemical and immunological reactions needed to generate accurate results. If the appropriate signal is displayed, analyze visual indicator in the detection zone to determine the presence or amount of analyte of interest in the sample.

The process allows the operator to determine if the sample has reached the detection and control zones of the device, whether or not the conditions are appropriate for generating accurate test results, and whether or not the sample contains an analyte of interest. Analyte-labeled receptor complexes that are captured by the immobilized binder may be capable of detection, either visually by the eye of the operator or via a measuring device. After an appropriate time, the operator may analyze the control zone visually or with the aid of a detection device to ascertain whether or not the assay has been completed, and then may ascertain the result of the assay by observing the detection zone. Certain steps in the method described above must naturally precede others for the invention to function as described. However, the invention is not limited to the order of the steps described if such order or sequence does not alter the functionality of the invention. That is, it is recognized that some steps may be performed before or after or in parallel with other steps without departing from the scope and spirit of the invention.

### Kits

The invention further provides kits for carrying out immunoassays utilizing the device as described herein. In one embodiment, a kit according to the invention may comprise the assay device with its incorporated reagents as well as a wicking solution and/or test sample pretreatment reagents. Other assay components known to one of ordinary skill in the art, such as buffers, stabilizers, detergents, bacteria inhibiting agents and the like may also be present in the kit. In addition, the kit may contain packaging materials and directions on how to use the device.

### EXAMPLES

The following example illustrates, but is not intended to limit the invention.

### α. Construction of Immunoassay Device

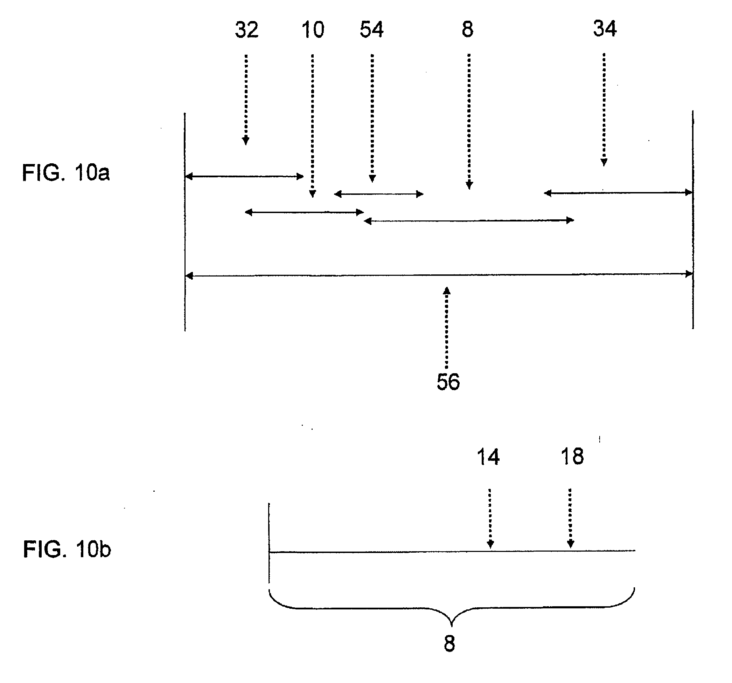

**FIGS. 10a****-b** illustrate representations of a lateral flow GAS immunoassay device constructed according to the invention. As shown in **FIG. 10a****,** a 15 mm sample pad **32** containing a dried down neutralization solution was juxtaposed with a tracer pad **10** with a length of 8 mm. The tracer pad **10** was juxtaposed with a 5 mm mixing pad **54,** which in turn was juxtaposed with a 25 mm nitrocellulose binder support medium **8.** The nitrocellulose binder support medium **8** was juxtaposed with a 17 mm sump pad **34.** As shown in **FIG. 10b****,** the nitrocellulose binder support medium **8** comprised a detection zone **14,** located 14 mm from the left side of the nitrocellulose binder support medium **8,** and a control zone **18,** located 20 mm from the left side of the nitrocellulose binder support medium **8.** All the above pads and medium were pressed onto a 60 mm laminate support **56** in the configuration shown in **FIG. 10a****.**

The detection zone **14** comprised a line of antibody directed against GAS antigen in a suitable buffer. The control zone **18** comprised a line of NAGBSA (Sigma A1034 or equivalent) in a suitable buffer. These lines were sprayed down by an applicator so that they were immobilized onto the nitrocellulose binder support medium **8.**

### b. Detection of GAS Antigen Under Varying Conditions

Three drops of extracted sample containing GAS antigen were applied to the sample pad via a dispense tube tip to the sample opening of the top of a device constructed as described above in Part (a). The sample was neutralized in the sample pad of the device. After neutralization the conditions were favorable to allow for binding of the GAS antigen and an antibody to the GAS antigen. The assays were then allowed to proceed. Signals in the control and detection zones were then visualized in the device through an aperture in the top of the device.

Three drops of an extracted sample not containing GAS antigen were applied to the sample pad via a dispense tube tip to the sample opening of the top of a second device constructed as described above in Part (a). The sample was neutralized in the sample pad of the device. After neutralization the conditions were favorable to allow for binding of the GAS antigen and an antibody to the GAS antigen. The assays were then allowed to proceed. Only a signal in the control zone was visualized in the device through an aperture in the top of the device.

Three drops of extracted sample containing GAS antigen were applied to the sample pad via a dispense tube tip to the sample opening of the top of a third device constructed as described above in Part (a). The sample was neutralized in the sample pad of the device. The assays were then allowed to proceed. Signals were not visualized in either the control or detection zone, indicating that either the sample did not reach the detection and control zone or that the conditions did not allow binding of the antigen or antibody to the detection or control zones. Because no signal was observed in the control zone, the lack of signal in the detection zone was deemed unreliable.

## Claims

1. An immunoassay device for determining the presence or amount of a bacterial carbohydrate in a sample comprising:
a binder support medium comprising a detection zone and a control zone;
wherein the detection zone comprises an immobilized binder capable of binding with the bacterial carbohydrate, and wherein the control zone is in fluid communication with the detection zone, the control zone comprising, in immobilized form, an analog of the bacterial carbohydrate, **characterized in that** the device tests for the presence or amount of a Group A Streptococcus bacterium, the bacterial carbohydrate comprises β-N-Acetyl-D-Glucosamine attached to a poly-rhamnose backbone, and the analog of the bacterial carbohydrate comprises Albumin, Bovine, p-Aminophenyl N-Acetyl β-D Glucosaminide.

2. The device of claim 1, wherein the device further comprises a labeled receptor comprising a receptor moiety and a label moiety, wherein the receptor moiety is capable of binding with the bacterial carbohydrate and the analog of the bacterial carbohydrate, and wherein the receptor moiety is attached to the label moiety.

3. The device of claim 2, further comprising a tracer pad, wherein the labeled receptor is located on said tracer pad.

4. The device of claim 1, further comprising a sump, wherein the sump is juxtaposed with the binder support medium.

5. The device of claim 1, further comprising a sample pad capable of accepting a sample suspected of containing the bacterial carbohydrate.

6. The device of claim 1, further comprising an upper housing and a lower housing.

7. The device of claim 1, further comprising an upper housing and a lower housing, wherein the upper housing comprises an aperture that provides fluid access to the binder support medium.

8. The device of claim 1, further comprising a neutralizing pad.

9. The device of claim 1, further comprising:
a. a sample pad capable of accepting a sample suspected of containing the bacterial carbohydrate;
b. an upper housing; and
c. a lower housing;
wherein the upper housing comprises an aperture that provides fluid access to the sample pad.

10. The device of claim 1, further comprising an upper housing and a lower housing wherein the upper housing comprises an aperture that provides visual access to the detection zone.

11. The device of claim 1, further comprising an upper housing and a lower housing wherein the upper housing comprises an aperture that provides visual access to the control zone.

12. An immunoassay kit for determining the presence or amount of a bacterial carbohydrate in a sample comprising:
a. a binder support medium comprising a detection zone and a control zone;
b. packaging materials;
wherein the detection zone comprises an immobilized binder, capable of binding with the bacterial carbohydrate, and wherein the control zone is in fluid communication with the detection zone, the control zone comprising, in immobilized form, an analog of the bacterial carbohydrate, **characterized in that** the kit tests for the presence or amount of a Group A Streptococcus bacterium, the bacterial carbohydrate comprises β-N-Acetyl-D-Glucosamine attached to a poly-rhamnose backbone, and wherein the analog of the bacterial carbohydrate comprises Albumin, Bovine, p-Aminophenyl N-Acetyl β-D Glucosaminide.

13. The kit of claim 12, further comprising a labeled receptor comprising a receptor moiety and a label moiety, wherein the receptor moiety is capable of binding with the bacterial carbohydrate and the analog of the bacterial carbohydrate, and wherein the receptor moiety is attached to the label moiety.

14. The kit of claim 12 further comprising a wicking solution.

15. The kit of claim 12 further comprising test sample pre-treatment reagents.

16. The kit of claim 12 further comprising instructions on how to use the kit.

17. A method of determining the presence or amount of an analyte of interest in a sample,
wherein the analyte of interest is a bacterial carbohydrate, the method comprising:
a. preparing a sample for application to an assay device;
b. applying the sample suspected of containing the analyte of interest to an assay device,
wherein the assay device comprises a labeled receptor;
c. binding the analyte of interest, if present in the sample, to the labeled receptor, thereby forming an analyte-labeled receptor complex, wherein the device provides an amount of unbound labeled receptor;
d. binding the analyte-labeled receptor complex, if present, to an immobilized binder capable of binding the analyte-labeled receptor complex in the sample, and incapable of binding the unbound labeled receptor, wherein the immobilized binder is located in a detection zone on a binder support medium;
e. binding the unbound labeled receptor to an immobilized analog of the bacterial carbohydrate, wherein the immobilized analog of the bacterial carbohydrate is located in a control zone on a binder support medium;
f. determining whether conditions in the assay device provide for accurate determination of the presence or amount of the bacterial carbohydrate in the sample by analyzing the control zone for a signal, wherein the presence of a signal verifies proper conditions; and
g. if the proper conditions are verified, determining the presence or amount of the bacterial carbohydrate in the sample by analyzing the detection zone for a signal, **characterized in that** the method tests for the presence or amount of a Group A Streptococcus bacterium, the bacterial carbohydrate comprises β-N-Acetyl-D-Glucosamine attached to a poly-rhamnose backbone, and the analog of the bacterial carbohydrate comprises Albumin, Bovine, p-Aminophenyl N-Acetyl β-D Glucosaminide.

18. A method of determining the presence or amount of a bacterial carbohydrate in a sample, the method comprising:
a. administering a liquid sample to an immunoassay device that comprises:
i. a labeled receptor comprising a receptor moiety and a label moiety, wherein the receptor moiety is capable of binding with the bacterial carbohydrate and wherein the receptor moiety is attached to the label moiety; and
ii. a binder support medium comprising a detection zone and a control zone;
iii. wherein the detection zone comprises an immobilized binder, capable of binding with the bacterial carbohydrate, and wherein the control zone is in fluid communication with the detection zone, the control zone comprising, in immobilized form, an analog of the bacterial carbohydrate that is capable of binding with the receptor moiety.
b. determining whether conditions in the immunoassay provide for accurate determination of the presence or amount of the bacterial carbohydrate t in the sample by analyzing the control zone for a signal, wherein the presence of a signal verifies proper conditions; and
c. if the proper conditions are verified, determining the presence or amount of the bacterial carbohydrate in the sample by analyzing the detection zone for a signal, **characterized in that** the method tests for the presence or amount of a Group A Streptococcus bacterium, the bacterial carbohydrate comprises β-N-Acetyl-D-Glucosamine attached to a poly-rhamnose backbone, and the analog of the bacterial carbohydrate comprises Albumin, Bovine, p-Aminophenyl N-Acetyl β-D Glucosaminide.

## Patentansprüche

1. Immunoassayvorrichtung zur Bestimmung der Anwesenheit oder Menge eines bakteriellen Kohlenhydrats in einer Probe, umfassend:
ein Bindemittelträgermedium, das eine Nachweiszone und eine Kontrollzone umfasst;
wobei die Nachweiszone ein immobilisiertes Bindemittel umfasst, das das bakterielle Kohlenhydrat binden kann, und wobei die Kontrollzone in Fluidverbindung mit der Nachweiszone steht, wobei die Kontrollzone in immobilisierter Form ein Analogon des bakteriellen Kohlenhydrats umfasst, **dadurch gekennzeichnet, dass** die Vorrichtung auf die Anwesenheit oder Menge eines Streptococcus-Bakteriums der Gruppe A testet, dass das bakterielle Kohlenhydrat an ein Polyrhamnosegerüst gebundenes β-N-Acetyl-D-glucosamin umfasst und dass das Analogon des bakteriellen Kohlenhydrats das p-Aminophenyl-N-acetyl-β-D-glucosaminid von Rinderalbumin umfasst.

2. Vorrichtung gemäß Anspruch 1, wobei die Vorrichtung weiterhin einen markierten Rezeptor umfasst, der eine Rezeptor-Struktureinheit und eine Marker-Struktureinheit umfasst, wobei die Rezeptor-Struktureinheit das bakterielle Kohlenhydrat und das Analogon des bakteriellen Kohlenhydrats binden kann und wobei die Rezeptor-Struktureinheit an die Marker-Struktureinheit gebunden ist.

3. Vorrichtung gemäß Anspruch 2, die weiterhin ein Indikatorfeld umfasst, wobei sich der markierte Rezeptor auf dem Indikatorfeld befindet.

4. Vorrichtung gemäß Anspruch 1, die weiterhin einen Sumpf umfasst, wobei der Sumpf neben dem Bindemittelträgermedium angeordnet ist.

5. Vorrichtung gemäß Anspruch 1, die weiterhin ein Probenfeld umfasst, das eine Probe aufnehmen kann, von der man annimmt, dass sie das bakterielle Kohlenhydrat enthalten könnte.

6. Vorrichtung gemäß Anspruch 1, die weiterhin ein Gehäuseoberteil und ein Gehäuseunterteil umfasst.

7. Vorrichtung gemäß Anspruch 1, die weiterhin ein Gehäuseoberteil und ein Gehäuseunterteil umfasst, wobei das Gehäuseoberteil eine Öffnung umfasst, die für einen Fluidzugang zum Bindemittelträgermedium sorgt.

8. Vorrichtung gemäß Anspruch 1, die weiterhin ein Neutralisationsfeld umfasst.

9. Vorrichtung gemäß Anspruch 1, die weiterhin Folgendes umfasst:
a. ein Probenfeld, das eine Probe aufnehmen kann, von der man annimmt, dass sie das bakterielle Kohlenhydrat enthalten könnte;
b. ein Gehäuseoberteil; und
c. ein Gehäuseunterteil;
wobei das Gehäuseoberteil eine Öffnung umfasst, die für einen Fluidzugang zum Probenfeld sorgt.

10. Vorrichtung gemäß Anspruch 1, die weiterhin ein Gehäuseoberteil und ein Gehäuseunterteil umfasst, wobei das Gehäuseoberteil eine Öffnung umfasst, die den Blick auf die Nachweiszone ermöglicht.

11. Vorrichtung gemäß Anspruch 1, die weiterhin ein Gehäuseoberteil und ein Gehäuseunterteil umfasst, wobei das Gehäuseoberteil eine Öffnung umfasst, die den Blick auf die Kontrollzone ermöglicht.

12. Immunoassaykit zur Bestimmung der Anwesenheit oder Menge eines bakteriellen Kohlenhydrats in einer Probe, umfassend:
a. ein Bindemittelträgermedium, das eine Nachweiszone und eine Kontrollzone umfasst;
b. Verpackungsmaterialien;
wobei die Nachweiszone ein immobilisiertes Bindemittel umfasst, das das bakterielle Kohlenhydrat binden kann, und wobei die Kontrollzone in Fluidverbindung mit der Nachweiszone steht, wobei die Kontrollzone in immobilisierter Form ein Analogon des bakteriellen Kohlenhydrats umfasst, **dadurch gekennzeichnet, dass** der Kit auf die Anwesenheit oder Menge eines Streptococcus-Bakteriums der Gruppe A testet, dass das bakterielle Kohlenhydrat an ein Polyrhamnosegerüst gebundenes β-N-Acetyl-D-glucosamin umfasst und dass das Analogon des bakteriellen Kohlenhydrats das p-Aminophenyl-N-acetyl-β-D-glucosaminid von Rinderalbumin umfasst.

13. Kit gemäß Anspruch 12, der weiterhin einen markierten Rezeptor umfasst, der eine Rezeptor-Struktureinheit und eine Marker-Struktureinheit umfasst, wobei die Rezeptor-Struktureinheit das bakterielle Kohlenhydrat und das Analogon des bakteriellen Kohlenhydrats binden kann und wobei die Rezeptor-Struktureinheit an die Marker-Struktureinheit gebunden ist.

14. Kit gemäß Anspruch 12, der weiterhin eine Vorbefeuchtungslösung umfasst.

15. Kit gemäß Anspruch 12, der weiterhin Testproben-Vorbehandlungsreagentien umfasst.

16. Kit gemäß Anspruch 12, der weiterhin eine Gebrauchsanweisung hierfür umfasst.

17. Verfahren zur Bestimmung der Anwesenheit oder Menge eines interessierenden Analyten in einer Probe, wobei es sich bei dem interessierenden Analyten um ein bakterielles Kohlenhydrat handelt, wobei das Verfahren Folgendes umfasst:
a. Vorbereiten einer Probe zur Auftragung auf eine Assayvorrichtung;
b. Auftragen der Probe, von der man annimmt, dass sie den interessierenden Analyten enthalten könnte, auf eine Assayvorrichtung, wobei die Assayvorrichtung einen markierten Rezeptor umfasst;
c. Binden des interessierenden Analyten, wenn er in der Probe vorhanden ist, an den markierten Rezeptor unter Bildung eines Komplexes aus Analyt und markiertem Rezeptor, wobei die Vorrichtung eine Menge an ungebundenem markiertem Rezeptor bereitstellt;
d. Binden des Komplexes aus Analyt und markiertem Rezeptor, falls vorhanden, an ein immobilisiertes Bindemittel, das den Komplex aus Analyt und markiertem Rezeptor in der Probe binden kann und den ungebundenen markierten Rezeptor nicht binden kann, wobei sich das immobilisierte Bindemittel in einer Nachweiszone auf einem Bindemittelträgermedium befindet;
e. Binden des ungebundenen markierten Rezeptors an ein immobilisiertes Analogon des bakteriellen Kohlenhydrats, wobei sich das immobilisierte Analogon des bakteriellen Kohlenhydrats in einer Kontrollzone auf einem Bindemittelträgermedium befindet;
f. Bestimmen, ob die Bedingungen in der Assayvorrichtung für eine fehlerfreie Bestimmung der Anwesenheit oder Menge des bakteriellen Kohlenhydrats in der Probe sorgen, indem man die Kontrollzone in Bezug auf ein Signal analysiert, wobei die Anwesenheit eines Signals einwandfreie Bedingungen bestätigt; und
g. wenn die einwandfreien Bedingungen bestätigt sind, Bestimmen der Anwesenheit oder Menge des bakteriellen Kohlenhydrats in der Probe durch Analysieren der Nachweiszone in Bezug auf ein Signal, **dadurch gekennzeichnet, dass** das Verfahren auf die Anwesenheit oder Menge eines Streptococcus-Bakteriums der Gruppe A testet, dass das bakterielle Kohlenhydrat an ein Polyrhamnosegerüst gebundenes β-N-Acetyl-D-glucosamin umfasst und dass das Analogon des bakteriellen Kohlenhydrats das p-Aminophenyl-N-acetyl-β-D-glucosaminid von Rinderalbumin umfasst.

18. Verfahren zur Bestimmung der Anwesenheit oder Menge eines bakteriellen Kohlenhydrats in einer Probe, wobei das Verfahren Folgendes umfasst:
a. Eingaben einer flüssigen Probe in eine Immunoassayvorrichtung, die Folgendes umfasst:
i. einen markierten Rezeptor, der eine Rezeptor-Struktureinheit und eine Marker-Struktureinheit umfasst, wobei die Rezeptor-Struktureinheit das bakterielle Kohlenhydrat binden kann und wobei die Rezeptor-Struktureinheit an die Marker-Struktureinheit gebunden ist; und
ii. ein Bindemittelträgermedium, das eine Nachweiszone und eine Kontrollzone umfasst;
iii. wobei die Nachweiszone ein immobilisiertes Bindemittel umfasst, das das bakterielle Kohlenhydrat binden kann, und
wobei die Kontrollzone in Fluidverbindung mit der Nachweiszone steht, wobei die Kontrollzone in immobilisierter Form ein Analogon des bakteriellen Kohlenhydrats umfasst, das an die Rezeptor-Struktureinheit binden kann;
b. Bestimmen, ob die Bedingungen in dem Immunassay für eine fehlerfreie Bestimmung der Anwesenheit oder Menge des bakteriellen Kohlenhydrats in der Probe sorgen, indem man die Kontrollzone in Bezug auf ein Signal analysiert, wobei die Anwesenheit eines Signals einwandfreie Bedingungen bestätigt; und
c. wenn die einwandfreien Bedingungen bestätigt sind, Bestimmen der Anwesenheit oder Menge des bakteriellen Kohlenhydrats in der Probe durch Analysieren der Nachweiszone in Bezug auf ein Signal, **dadurch gekennzeichnet, dass** das Verfahren auf die Anwesenheit oder Menge eines Streptococcus-Bakteriums der Gruppe A testet, dass das bakterielle Kohlenhydrat an ein Polyrhamnosegerüst gebundenes β-N-Acetyl-D-glucosamin umfasst und dass das Analogon des bakteriellen Kohlenhydrats das p-Aminophenyl-N-acetyl-β-D-glucosaminid von Rinderalbumin umfasst.

## Revendications

1. Dispositif de dosage immunologique pour déterminer la présence ou la quantité d'un glucide bactérien dans un échantillon comprenant :
un milieu de support de liant comprenant une zone de détection et une zone de contrôle :
où la zone de détection comprend un liant immobilisé capable de se lier avec le glucide bactérien et où la zone de contrôle est en communication liquide avec la zone de détection, la zone de contrôle comprenant, sous forme immobilisée, un analogue du glucide bactérien, **caractérisé en ce que** le dispositif teste pour la présence ou la quantité d'une bactérie Streptococcus du Groupe A, le glucide bactérien comprend de la β-N-acétyl-D-glucosamine fixée à un squelette de poly-rhamnose, et l'analogue du glucide bactérien comprend l'albumine, p-aminophényl-N-acétyl-β-D-glucosaminide bovine.

2. Dispositif selon la revendication 1, où le dispositif comprend en outre un récepteur marqué comprenant un radical de récepteur et un radical de marqueur, où le radical de marqueur est capable de se lier avec le glucide bactérien et l'analogue du glucide bactérien, et où le radical de récepteur est fixé au radical de marqueur.

3. Dispositif selon la revendication 2, comprenant en outre un tapis traceur, où le récepteur marqué est localisé sur ledit tapis traceur.

4. Dispositif selon la revendication 1, comprenant en outre un réservoir, où le réservoir est juxtaposé avec le milieu de support de liant.

5. Dispositif selon la revendication 1, comprenant en outre un tapis d'échantillon capable d'accepter un échantillon suspecté de contenir le glucide bactérien.

6. Dispositif selon la revendication 1, comprenant en outre un logement supérieur et un logement inférieur.

7. Dispositif selon la revendication 1, comprenant en outre un logement supérieur et un logement inférieur, où le logement supérieur comprend une ouverture qui permet l'accès des liquides au milieu de support de liant.

8. Dispositif selon la revendication 1, comprenant en outre un tapis de neutralisation.

9. Dispositif selon la revendication 1, comprenant en outre :
a. un tapis d'échantillon capable d'accepter un échantillon suspecté de contenir le glucide bactérien ;
b. un logement supérieur ; et
c. un logement inférieur ;
où le logement supérieur comprend une ouverture qui permet l'accès des liquides au tapis d'échantillon.

10. Dispositif selon la revendication 1, comprenant en outre un logement supérieur et un logement inférieur où le logement supérieur comprend une ouverture qui fournit un accès visuel à la zone de détection.

11. Dispositif selon la revendication 1, comprenant en outre un logement supérieur et un logement inférieur où le logement supérieur comprend une ouverture qui fournit un accès visuel à la zone de contrôle.

12. Kit de dosage immunologique pour déterminer la présence ou la quantité d'un glucide bactérien dans un échantillon comprenant :
a. un milieu de support de liant comprenant une zone de détection et une zone de contrôle ;
b. des matériaux de conditionnement ;
où la zone de détection comprend un liant immobilisé, capable de se lier avec le glucide bactérien, et où la zone de contrôle est en communication liquide avec la zone de détection, la zone de contrôle comprenant, sous forme immobilisée, un analogue du glucide bactérien, **caractérisé en ce que** le kit teste pour la présence ou la quantité d'une bactérie Streptococcus du Groupe A, le glucide bactérien comprend de la β-N-acétyl-D-glucosamine fixée à un squelette de poly-rhamnose, et où l'analogue du glucide bactérien comprend l'albumine, p-aminophényl-N-acétyl-β-D-glucosaminide bovine.

13. Kit selon la revendication 12, comprenant en outre un récepteur marqué comprenant un radical de récepteur et un radical de marqueur, où le radical de récepteur est capable de se lier avec le glucide bactérien et l'analogue du glucide bactérien, et où le radical de récepteur est fixé au radical de marqueur.

14. Kit selon la revendication 12, comprenant en outre une solution d'absorption capillaire.

15. Kit selon la revendication 12, comprenant en outre des réactifs de prétraitement des échantillons à tester.

16. Kit selon la revendication 12, comprenant des instructions sur comment utiliser le kit.

17. Procédé de détermination de la présence ou de la quantité d'un analyte d'intérêt dans un échantillon, où l'analyte d'intérêt est un glucide bactérien, le procédé comprenant :
a. la préparation d'un échantillon pour une application sur un dispositif de dosage ;
b. l'application de l'échantillon suspecté de contenir l'analyte d'intérêt sur un dispositif de dosage où le dispositif de dosage comprend un récepteur marqué ;
c. la liaison de l'analyte d'intérêt, s'il est présent dans l'échantillon, au récepteur marqué, formant de cette manière un complexe analyte-récepteur marqué, où le dispositif fournit une quantité de récepteur marqué non lié ;
d. la liaison du complexe analyte-récepteur marqué, s'il est présent, à un liant immobilisé capable de lier le complexe analyte-récepteur marqué dans l'échantillon, et incapable de lier le récepteur marqué non lié, où le liant immobilisé est localisé dans une zone de détection sur un milieu de support de liant ;
e. la liaison du récepteur marqué non lié à un analogue immobilisé du glucide bactérien, où l'analogue immobilisé du glucide bactérien est localisé dans une zone de contrôle sur un milieu de support de liant ;
f. la détermination si les conditions dans le dispositif de dosage permettent une détermination exacte de la présence ou de la quantité du glucide bactérien dans l'échantillon en analysant la zone de contrôle pour un signal, où la présence d'un signal vérifie des conditions correctes ; et
g. si les conditions correctes sont vérifiées, la détermination de la présence ou de la quantité du glucide bactérien dans l'échantillon en analysant la zone de détection pour un signal, **caractérisé en ce que** le procédé teste pour la présence ou la quantité d'une bactérie Streptococcus du Groupe A, le glucide bactérien comprend de la β-N-acétyl-D-glucosamine fixée à un squelette de poly-rhamnose, et l'analogue du glucide bactérien comprend l'albumine, p-aminophényl-N-acétyl-β-D-glucosaminide bovine.

18. Procédé de détermination de la présence ou de la quantité d'un glucide bactérien dans un échantillon, le procédé comprenant :
a. l'administration d'un échantillon liquide à un dispositif de dosage immunologique qui comprend :
i. un récepteur marqué comprenant un radical de récepteur et un radical de marqueur, où le radical de récepteur est capable de se lier avec le glucide bactérien et où le radical de récepteur est fixé au radical de marqueur ; et
ii. un milieu de support de liant comprenant une zone de détection et une zone de contrôle ;
iii. où la zone de détection comprend un liant immobilisé, capable de se lier avec le glucide bactérien, et où la zone de contrôle est en communication liquide avec la zone de détection, la zone de contrôle comprenant, sous forme immobilisée, un analogue du glucide bactérien qui est capable de se lier avec le radical de récepteur.
b. la détermination si les conditions dans le dosage immunologique permettent une détermination exacte de la présence ou de la quantité du glucide bactérien dans l'échantillon en analysant la zone de contrôle pour un signal, où la présence d'un signal vérifie des conditions correctes ; et
c. si les conditions correctes sont vérifiées, la détermination de la présence ou de la quantité du glucide bactérien dans l'échantillon en analysant la zone de détection pour un signal, **caractérisé en ce que** le procédé teste pour la présence ou la quantité d'une bactérie Streptococcus du Groupe A, le glucide bactérien comprend de la β-N-acétyl-D-glucosamine fixée à un squelette de poly-rhamnose, et l'analogue du glucide bactérien comprend l'albumine, p-aminophényl-N-acétyl-β-D-glucosaminide bovine.
